Europäisches Patentamt

European Patent Office

Office européen des brevets

(19)

(11) Numéro de publication: **0 271 371 B1**

(12) **FASCICULE DE BREVET EUROPEEN**

(45) Date de publication de fascicule du brevet:
18.09.91

(51) Int. Cl.5: **C07C 57/04**, C07C 53/124,
C07C 51/48

(21) Numéro de dépôt: 87402374.0

(22) Date de dépôt: 22.10.87

(54) Procédé de séparation extractive d'un acide carboxylique à partir d'une solution aqueuse dudit acide.

(30) Priorité: 14.11.86 FR 8615840

(43) Date de publication de la demande:
15.06.88 Bulletin 88/24

(45) Mention de la délivrance du brevet:
18.09.91 Bulletin 91/38

(84) Etats contractants désignés:
AT BE CH DE ES FR GB GR IT LI LU NL SE

(56) Documents cités:
FR-A- 716 448
FR-A- 2 383 160

(73) Titulaire: ATOCHEM
4 & 8, Cours Michelet La Défense 10
F-92800 Puteaux(FR)

(72) Inventeur: Samuel, Yves
38 les Coccinelles
F-57500 St Avold(FR)

(74) Mandataire: Rochet, Michel et al
ATOCHEM Département Propriété Industrielle La Défense 10 Cédex 42
F-92091 Paris La Défense(FR)

## Description

Il est bien connu de préparer l'acide méthacrylique par oxydation catalytique en phase gazeuse de l'isobutylène, du tert-butanol ou de la méthacroléïne, ou bien encore par déshydrogénation oxydante de l'acide isobutyrique. Dans un cas comme dans l'autre, en général le gaz réactionnel absorbe de l'eau et devient une solution aqueuse d'acide méthacrylique. Cette solution contient généralement, quelque soit le procédé utilisé, de faibles quantités d'impuretés et/ou de co-produits qu'il est souhaitable d'éliminer en vue d'obtenir de l'acide méthacrylique purifié. L'acide méthacrylique purifié peut être obtenu par extraction en utilisant un solvant organique approprié puis en procédant à la distillation de la solution extraite. Il est bien évidemment souhaitable qu'une proportion aussi élevée que possible d'impuretés et/ou de co-produits reste dans la solution aqueuse, lors de la mise en oeuvre du procédé d'extraction par un solvant organique.

Pour résoudre le problème ainsi défini, on a déjà proposé différentes solutions. Ainsi le brevet FR-A-2.418.216 décrit un procédé dans lequel on extrait l'acide méthacrylique à l'aide d'un solvant mélangé contenant de 15 à 50 % en poids d'ester méthacrylique dans un solvant organique dont le point d'ébullition est supérieur à 120°C, on sépare par distillation l'ester méthacrylique et la plus grande partie du deuxième solvant d'une part, une solution d'acide méthacrylique dans le reste du solvant d'autre part. Ce procédé présente le grave inconvénient de ne pas permettre le recyclage du solvant mélangé étant donné que, à temps de séjour infini, l'ester méthacrylique est susceptible de s'oligomériser ou se polymériser. Le brevet FR-A-2.451.360 décrit un procédé dans lequel l'acide méthacrylique est extrait à l'aide d'un mélange d'une cétone et d'un solvant aromatique afin de rejeter l'eau et les impuretés. La distillation du solvant riche donne l'acide méthacrylique contenant de l'acide acétique, tandis que l'eau et le solvant obtenus en tête de colonne sont traités pour récupérer le solvant. Le système d'extraction proposé par ce document présente l'inconvénient d'une sélectivité insuffisante pour l'acide méthacrylique et d'une trop forte solubilité dans l'eau.

Le brevet japonais n° 1.037.851 décrit un système d'extraction de l'acide méthacrylique, ce système présentant une haute sélectivité pour l'acide méthacrylique et une faible solubilité dans l'eau et étant constitué de :

A) au moins un ester acétique d'un alcool aliphatique ayant de 2 à 4 atomes de carbone, ledit ester présentant un coefficient de partage élevé pour l'acide méthacrylique et une solubilité élevée dans l'eau, et

B) au moins un hydrocarbure choisi dans le groupe comprenant l'hexane, l'heptane et le benzène, ledit hydrocarbure présentant un coefficient de partage médiocre pour l'acide méthacrylique et une faible solubilité dans l'eau, le rapport en poids A/B étant de préférence compris entre 0,05 et 1,5.

Toutefois le procédé d'extraction proposé par ce document présente l'inconvénient de nécessiter un rapport pondéral du système solvant à l'acide méthacrylique égal à 4,9. Un tel rapport est très important et implique d'une part l'investissement d'appareils d'extraction de grandes dimensions et d'autre part des consommations énergétiques élevées.

Le problème que la présente invention vise à résoudre consiste donc à mettre au point un procédé de séparation extractive de l'acide méthacrylique, à partir d'une solution aqueuse d'acide méthacrylique contenant des impuretés, qui puisse être mis en oeuvre de manière efficace sans nécessiter les investissements importants des procédés antérieurement connus et tout en réduisant les consommations énergétiques. Par ailleurs, dans le cadre des études ayant conduit à la présente invention, on a pu constater que la solution proposée s'applique aussi à la séparation extractive de l'acide isobutyrique, seul ou en mélange avec l'acide méthacrylique.

L'objet de la présente invention est donc un procédé de séparation d'au moins un acide choisi parmi l'acide isobutyrique et l'acide méthacrylique par extraction à partir d'une solution aqueuse dudit acide en utilisant un système solvant organique comprenant du toluène, suivie d'une distillation au cours de laquelle le système solvant est récupéré et renvoyé inté gralement à l'extraction, caractérisé en ce que ledit système solvant est un mélange de toluène et d'au moins un ester organique saturé faiblement soluble dans l'eau et ayant une température d'ébullition comprise entre 98°C et 130°C. Des exemples non limitatifs d'esters organiques saturés conformes à la présente invention sont l'acétate d'isobutyle, l'acétate de n-butyle, le propionate d'éthyle, le propionate de propyle et l'isobutyrate d'éthyle. Selon un mode de réalisation particulier de la présente invention, le rapport pondéral du toluène à l'ester organique saturé est de préférence compris entre 0,5 et 2. Selon un autre mode de réalisation de la présente invention, le système solvant est utilisé dans un rapport pondéral à l'acide au plus égal à 2,5. De plus l'étape d'extraction du procédé selon l'invention peut être mise en oeuvre à une température quelconque choisie entre +10°C et +50°C et sous une pression quelconque choisie entre 1 et 8 bars.

La plupart des méthodes d'extraction (discontinue, continue à contre-courant, continue à étages) sont applicables industriellement dans le cadre du procédé selon l'invention. La méthode continue à contre-courant peut être considérée comme la plus avantageuse, la solution d'acide étant alors alimentée en tête de la colonne d'extraction tandis que le système solvant est alimenté en pied de ladite colonne.

L'acide purifié à l'issue de l'étape d'extraction du procédé selon l'invention est aisément obtenu par distillation ordinaire. Celle-ci sera effectuée dans des conditions propres à éviter la polymérisation de l'acide lorsqu'il s'agit de l'acide méthacrylique. De telles conditions peuvent être remplies en opérant sous pression réduite (c'est-à-dire inférieure à la pression atmosphérique) et/ou en présence d'au moins un inhibiteur de polymérisation.

Le système solvant utilisé dans le cadre du procédé selon l'invention procure de nombreux avantages :

- il possède un bon pouvoir d'extraction même lorsque l'acide carboxylique est assez fortement dilué dans l'eau,
- il permet d'éviter une concentration trop élevée en eau dans l'extrait, qui poserait des problèmes dans les étapes ultérieures de séparation en raison de l'azéotrope formé non seulement avec l'un ou l'autre des solvants organiques mais aussi avec l'acide carboxylique (lors de la distillation du solvant, une teneur importante en eau crée un entraînement d'acide carboxylique dans le solvant recyclé, ce qui réduit la qualité de l'extraction),
- en raison de la température d'ébullition de l'ester organique saturé, l'acide acétique éventuellement présent dans l'extrait (notamment lorsque l'acide méthacrylique a été obtenu à partir de la méthacroléïne ou de l'acide isobutyrique) pourra être convenablement recyclé à l'extraction, de façon à ce que l'acide carboxylique purifié soit exempt d'acide acétique.
- en raison du caractère saturé de l'ester organique associé au toluène dans le système solvant, il est exclu que ledit ester puisse s'oligomériser ou se polymériser au cours des recyclages successifs du système solvant.

Le procédé selon l'invention permet de séparer la quasi-totalité de l'acide carboxylique contenu dans la solution aqueuse tout en utilisant des quantités de système solvant nettement plus modérées que celles connues d'après l'art antérieur cité précédemment et, par conséquent, tout en mettant en jeu de moindres consommations énergétiques et des appareils d'extraction de moindres dimensions, donc des investissements plus faibles. Vu sous ce double aspect, le procédé selon la présente invention présente un net avantage économique par rapport à l'état de la technique.

Les exemples suivants sont donnés à titre illustratif et non limitatif de la présente invention (tous les pourcentages sont donnés en poids).

## EXEMPLE 1

Un mélange comprenant 23,56 kg/h d'eau, 1,66 kg/h d'acide isobutyrique et 4,29 kg/h d'acide méthacrylique est extrait avec 7,65 kg/h d'un système solvant comprenant 47 % d'acétate d'isobutyle et 53 % de toluène. L'extraction a lieu en continu dans un dispositif comprenant 8 étages d'extraction, chaque étage comprenant un mélangeur et un décanteur. La température est de 21,5 °C.

Le raffinat ne comprend que 0,01 kg/h d'acide isobutyrique et 0,04 kg/h d'acide méthacrylique, ce qui correspond à un rendement d'extraction de 99 %. L'extrait contient 3,7 % d'eau seulement.

## EXEMPLE 2 (comparatif)

On répète la procédure de l'exemple 1, sauf que le solvant est du toluène pur. Le rendement d'extraction en acide est de 90 % seulement (le raffinat comprend 0,16 kg/h d'acide isobutyrique et 0,44 kg/h d'acide méthacrylique).

## EXEMPLE 3 (comparatif)

On répète la procédure de l'exemple 1, sauf que le solvant est de l'acétate d'isobutyle pur. Le rendement d'extraction est alors de 98 %. Cependant la teneur en eau de l'extrait est de 6,5 %.

## EXEMPLE 4

On répète la procédure d'extraction de l'exemple 1, tout en remplaçant l'acétate d'isobutyle par l'isobutyrate d'éthyle. Le résultat obtenu est identique à celui mentionné à l'exemple 1.

## EXEMPLE 5 (comparatif)

On répète la procédure d'extraction de l'exemple 3, tout en remplaçant l'acétate d'isobutyle par l'isobutyrate d'éthyle. Le résultat obtenu est identique à celui mentionné à l'exemple 3.

## Revendications

1. Procédé de séparation d'au moins un acide choisi parmi l'acide isobutyrique et l'acide méthacrylique par extraction à partir d'une solution aqueuse dudit acide en utilisant un système solvant organique comprenant du toluène, suivie d'une distillation au cours de laquelle le système solvant est récupéré et renvoyé intégralement à l'extraction, caractérisé en ce que ledit système solvant est un mélange de toluène et d'au moins un ester organique saturé faiblement soluble dans l'eau et ayant une température d'ébullition comprise entre 98°C et 130°C.

2. Procédé selon la revendication 1, caractérisé en ce que l'ester organique saturé est choisi parmi l'acétate d'isobutyle, l'acétate de n-butyle, le proprionate d'éthyle, le propionate de propyle et l'isobutyrate d'éthyle.

3. Procédé selon l'une des revendications 1 et 2, caractérisé en ce que le rapport pondéral du toluène à l'ester organique saturé est compris entre 0,5 et 2.

4. Procédé selon l'une des revendications 1 à 3, caractérisé en ce que le système solvant est utilisé dans un rapport pondéral à l'acide au plus égal à 2,5.

5. Procédé selon l'une des revendications 1 à 4, caractérisé en ce que l'étape d'extraction est mise en oeuvre à une température choisie entre +10°C et +50°C.

6. Procédé selon l'une des revendications 1 à 5, caractérisé en ce que l'étape d'extraction est mise en oeuvre sous une pression comprise entre 1 et 8 bars.

7. Procédé selon l'une des revendications 1 à 6, caractérisé en ce que l'étape de distillation est effectuée dans des conditions propres à éviter la polymérisation de l'acide.

8. Procédé selon la revendication 7, caractérisé en ce que la distillation est effectuée sous pression réduite.

9. Procédé selon l'une des revendications 7 et 8, caractérisé en ce que la distillation est effectuée en présence d'au moins un inhibiteur de polymérisation.

10. Procédé selon l'une des revendications 1 à 9, caractérisé en ce que l'extraction est effectuée selon la méthode continue à contre-courant.

## Claims

1. Process for the separation of at least one acid chosen from isobutyric acid and methacrylic acid by extraction from an aqueous solution of the said acid using an organic solvent system containing toluene, followed by a distillation during which the solvent system is recovered and returned in its entirety to the extraction, characterised in that the said solvent system is a mixture of toluene and at least one saturated organic ester with a low water solubility and having a boiling point of between 98°C and 130°C.

2. Process according to Claim 1, characterised in that the saturated organic ester is chosen from among isobutyl acetate, n-butyl acetate, ethyl propionate, propyl propionate and ethyl isobutyrate.

3. Process according to either of Claims 1 and 2, characterised in that the gravimetric ratio of toluene to saturated organic ester is between 0.5:1 and 2:1.

4. Process according to one of Claims 1 to 3, characterised in that the solvent system is employed in a gravimetric ratio relative to the acid which is at most equal to 2.5:1.

5. Process according to one of Claims 1 to 4, characterised in that the extraction stage is carried out at a temperature chosen between +10°C and +50°C.

6. Process according to one of Claims 1 to 5, characterised in that the extraction stage is carried out at a pressure of between 1 and 8 bars.

7. Process according to one of Claims 1 to 6, characterised in that the distillation stage is carried out under conditions suitable for preventing the polymerisation of the acid.

8. Process according to Claim 7, characterised in that the distillation is carried out under reduced pressure.

9. Process according to either of Claims 7 and 8, characterised in that the distillation is carried out in the presence of at least one polymerisation inhibitor.

10. Process according to one of Claims 1 to 9, characterised in that the extraction is carried out according to a continuous countercurrent method.

## Patentansprüche

1. Verfahren zum Abtrennen mindestens einer Säure aus der Gruppe bestehend aus Isobuttersäure und Methacrylsäure durch Extraktion aus einer wäßrigen Lösung dieser Säure unter Verwendung eines Toluol enthaltenden organischen Lösungsmittelsystems, gefolgt von einer Destillation, im Laufe welcher das Lösungsmittelsystem rückgewonnen und in vollem Umfang zur Extraktion zurückgeschickt wird, dadurch gekennzeichnet, daß das genannte Lösungsmittelsystem eine Mischung aus Toluol und mindestens einem gesättigten organischen Ester ist, der im Wasser schwach löslich ist und eine Siedetemperatur zwischen 98° C und 130° C besitzt.

2. Verfahren nach Anspruch 1, dadurch gekennzeichnet, daß der gesättigte organische Ester ausgewählt wird aus der Gruppe bestehend aus Isobutylacetat, n-Butylacetat, Ethylpropionat, Propylpropionat und Ethylisobutyrat.

3. Verfahren nach Anspruch 1 oder 2, dadurch gekennzeichnet, daß das Gewichtsverhältnis zwischen dem Toluol und dem gesättigten organischen Ester zwischen 0,5 und 2 beträgt.

4. Verfahren nach einem der Ansprüche 1 bis 3, dadurch gekennzeichnet, daß das Lösungsmittelsystem in einem Gewichtsverhältnis zur Säure von höchstens 2,5 eingesetzt wird.

5. Verfahren nach einem der Ansprüche 1 bis 4, dadurch gekennzeichnet, daß der Extraktionsschritt bei einer zwischen +10° C und +50° C ausgewählten Temperatur durchgeführt wird.

6. Verfahren nach einem der Ansprüche 1 bis 5, dadurch gekennzeichnet, daß der Extraktionsschritt unter einem Druck zwischen 1 und 8 bar durchgeführt wird.

7. Verfahren nach einem der Ansprüche 1 bis 6, dadurch gekennzeichnet, daß der Destillationsschritt unter Bedingungen durchgeführt wird, die geeignet sind, die Polymerisation der Säure zu verhindern.

8. Verfahren nach Anspruch 7, dadurch gekennzeichnet, daß die Destillation unter reduziertem Druck durchgeführt wird.

9. Verfahren nach Anspruch 7 oder 8, dadurch gekennzeichnet, daß die Destillation in Gegenwart mindestens eines Polymerisationsinhibitors durchgeführt wird.

10. Verfahren nach einem der Ansprüche 1 bis 9, dadurch gekennzeichnet, daß die Extraktion kontinuierlich im Gegenstrom durchgeführt

wird.